# EUROPEAN PATENT SPECIFICATION

(11) **EP 2 857 391 B1**
(45) Date of publication and mention of the grant of the patent: **19.07.2017**
(21) Application number: 12878365.1
(22) Date of filing: 04.06.2012
(51) Int. Cl.: C07D 307/60, C07D 307/58, C07D 307/28, C07D 307/20, C07C 61/35, C07C 62/32, C07C 62/36, C07C 62/38, C07C 51/42, A61K 31/365, A61K 31/341, A61K 31/19, A61K 31/194, A61P 25/28, A61P 25/16, A61P 25/14, A61P 25/00

(54) **LABDANE DITERPENOID COMPOUNDS, SEMEN BIOTAE EXTRACT, AND PREPARATION METHOD AND USE THEREOF**
LABDAN-DITERPENOID-VERBINDUNGEN, SEMEN BIOTAE-EXTRAKT UND HERSTELLUNGSVERFAHREN UND VERWENDUNG DAVON
COMPOSÉS DITERPÉNOÏDES DE LABDANE, EXTRAIT DE SEMEN BIOTAE ET LEUR PROCÉDÉ DE PRÉPARATION ET LEUR UTILISATION

(43) Date of publication of application: 08.04.2015
(73) Proprietor: Sun Yat-Sen University, Guangzhou, Guangdong 510275 (CN)
(72) Inventor: SU, Weiwei, Guangzhou Guangdong 510275 (CN); WANG, Yonggang, Guangzhou Guangdong 510275 (CN); LIANG, Fengyin, Guangzhou Guangdong 510275 (CN); WANG, Ning, Guangzhou Guangdong 510275 (CN); PEI, Zhong, Guangzhou Guangdong 510275 (CN); LIU, Haibin, Guangzhou Guangdong 510275 (CN)
(74) Representative: Sun, Yiming
(86) International application number: PCT/CN2012/076443
(87) International publication number: WO 2013/181786

(56) References cited:
- US-A1- 2004 057 908
- US-A1- 2004 057 908
- X.-Y. REN ET AL: "Labdane diterpenes from the seeds of Platycladus orientalis", JOURNAL OF ASIAN NATURAL PRODUCTS RESEARCH, vol. 8, no. 8, 1 December 2006 (2006-12-01), pages 677-682, XP055175752, ISSN: 1028-6020, DOI: 10.1080/10286020500246584
- WANG ET AL: "Diterpenoids from the pericarp of Platycladus orientalis", PHYTOCHEMISTRY, vol. 69, no. 2, 1 January 2008 (2008-01-01), pages 518-526, XP022408916, PERGAMON PRESS, GB ISSN: 0031-9422, DOI: 10.1016/J.PHYTOCHEM.2007.07.023
- KUO ET AL: "Chemical Constituents of the Pericarp of Platycladus Orientalis", JOURNAL OF THE CHINESE CHEMICAL SOCIETY (TAIPEI), vol. 46, no. 5, 1 January 1999 (1999-01-01), pages 819-824, XP055175755,
- K A KOO ET AL: "Pinusolide and 15-methoxypinusolidic acid attenuate the neurotoxic effect of staurosporine in primary cultures of rat cortical cells", BRITISH JOURNAL OF PHARMACOLOGY, vol. 150, no. 1, 1 January 2007 (2007-01-01), pages 65-71, XP055175756, ISSN: 0007-1188, DOI: 10.1038/sj.bjp.0706944
- INOUE M ET AL: "Terpenoids from the seed of platycladus orientalis", PHYTOCHEMISTRY, vol. 24, no. 7, 1 January 1985 (1985-01-01), pages 1602-1604, XP026680023, PERGAMON PRESS, GB ISSN: 0031-9422, DOI: 10.1016/S0031-9422(00)81075-4 [retrieved on 1985-01-01]
- KUO ET AL: "Four New Terpenes from the Pericarp of Platycladus orientalis", CHEM. PHARM. BULL., vol. 48, no. 6, 1 January 2000 (2000-01-01), pages 766-768, XP055175759,
- JAVAD ASILI ET AL: "Labdanes and Isopimaranes from Platycladus orientalis and Their Effects on Erythrocyte Membrane and on Plasmodium f alciparum Growth in the Erythrocyte Host Cells", JOURNAL OF NATURAL PRODUCTS, vol. 67, no. 4, 1 April 2004 (2004-04-01), pages 631-637, XP055175764, ISSN: 0163-3864, DOI: 10.1021/np034033e
- S. STARK ET AL: "Composition of lipophilic compounds and carbohydrates in the accumulated plant litter and soil organic matter in boreal forests", EUROPEAN JOURNAL OF SOIL SCIENCE, vol. 63, no. 1, 13 December 2011 (2011-12-13), pages 65-74, XP055175776, ISSN: 1351-0754, DOI: 10.1111/j.1365-2389.2011.01411.x
- DATABASE CAPLUS [Online] CHEMICAL ABSTRACTS SERVICE, COLUMBUS, OHIO, US; 25 February 2010 (2010-02-25), ALBERTE, RANDALL S. ET AL: "Anti-inflammatory and anti-allergy extracts from nettle", XP055191708, retrieved from STN Database accession no. 2010:55352 & US 2010/009927 A1 (ALBERTE, RANDALL S. ET AL) 14 January 2010 (2010-01-14)
- LI C J ET AL: "Bis-sesquiterpenes and diterpenes from Chloranthushenryi", PHYTOCHEMISTRY, vol. 69, no. 16, 1 November 2008 (2008-11-01), pages 2867-2874, XP025685618, PERGAMON PRESS, GB ISSN: 0031-9422, DOI: 10.1016/J.PHYTOCHEM.2008.08.022 [retrieved on 2008-10-15]
- CHI-I CHANG ET AL: "A new labdane-type diterpene from the bark of Juniperus chinensis Linn.", NATURAL PRODUCT RESEARCH, vol. 22, no. 13, 10 September 2008 (2008-09-10), pages 1158-1162, XP055175779, ISSN: 1478-6419, DOI: 10.1080/14786410601132444
- ALVAREZ-MANZANEDA ROLDAN, ENRIQUE ET AL: "A New Route toward 7-Oxo-13-hydroxy-8,11,13-podocarpatrienes from Labdane Diterpenes", JOURNAL OF NATURAL PRODUCTS, vol. 69, no. 4, 2006, pages 563-566, XP002750765, DOI: 10.1021/NP0502847
- WEN-CHIUNG S ET AL: "Labdanes from Cryptomeria japonica", PHYTOCHEMISTRY, vol. 37, no. 4, 7 November 1994 (1994-11-07), pages 1109-1114, XP026685839, PERGAMON PRESS, GB ISSN: 0031-9422, DOI: 10.1016/S0031-9422(00)89538-2 [retrieved on 1994-11-07]
- RODRIGUES-FILHO ET AL: "Hydroxylation of the Labdane Diterpene Cupressic Acid by Fusarium graminearum", JOURNAL OF THE BRAZILIAN CHEMICAL SOCIETY, vol. 13, no. 2, 1 January 2002 (2002-01-01), pages 266-269, XP055175790,
- NIE JING ET AL: "Comparison of Modified CoMFAs in Study of PAF Antagonist", HUAXUE XUEBAO : YUEKAN = ACTA CHIMICA SINICA, KEXUE CHUBANSHE, CN, vol. 61, no. 7, 1 January 2003 (2003-01-01), pages 1129-1135, XP008175459, ISSN: 0567-7351
- BARRERO A F ET AL: "Biomimetic synthesis of 12-oxy-pimaranes from 12,13-epoxy-labdadienes", TETRAHEDRON, vol. 47, no. 25, 1 January 1991 (1991-01-01), pages 4441-4456, XP026614947, ELSEVIER SCIENCE PUBLISHERS, AMSTERDAM, NL ISSN: 0040-4020, DOI: 10.1016/S0040-4020(01)87113-1 [retrieved on 1991-01-01]
- CALDERON J S ET AL: "Labdane diterpenes from Brickellia glomerata", PHYTOCHEMISTRY, vol. 26, no. 9, 1 January 1987 (1987-01-01), pages 2639-2641, XP026605097, PERGAMON PRESS, GB ISSN: 0031-9422, DOI: 10.1016/S0031-9422(00)83897-2 [retrieved on 1987-01-01]
- CAPUTO ET AL: "XXXII/ Diterpenoids of Araucariaceae. VII. Minor Diterpenes from e Resin of Araucaria Imbricata", GAZZETTA CHIMICA ITALIANA, vol. 106, no. 11-12, 1 January 1976 (1976-01-01), pages 1119-1121, XP008175458, SOCIET GBP A CHIMICA ITALIANA, IT ISSN: 0016-5603
- J S MILLS ET AL: "REVISED STRUCTURE OF METHYL SCIADOPATE", TETRAHEDRON, vol. 30, no. 22, 1 January 1974 (1974-01-01), pages 4021-4023, XP055175831,
- KOO, KYUNG AH ET AL: "15-Methoxypinusolidic acid from Biota orientalis attenuates glutamate-induced neurotoxicity in primary cultured rat cortical cells", TOXICOLOGY IN VITRO, vol. 20, no. 6, 2006, pages 936-941, XP002750766, DOI: 10.1016/J.TIV.2006.02.001
- DATABASE CAPLUS [Online] CHEMICAL ABSTRACTS SERVICE, COLUMBUS, OHIO, US; KOO, GYEONG A. ET AL: "Preventing and therapeutic agent containing pinusolide derivative as effective ingredient for neurodegenerative disease", XP002750767, retrieved from STN Database accession no. 2004:980735 -& KR 2003 056 771 A (ELCOM SCIENCE CO., LTD., S. KOREA) 4 July 2003 (2003-07-04)
- ABDEL-SATTAR, ESSAM ET AL: "Chemical and biological investigation of Araucaria heterophylla Salisb. resin", ZEITSCHRIFT FUER NATURFORSCHUNG, C: JOURNAL OF BIOSCIENCES, vol. 64, no. 11/12, 2009, pages 819-823, XP002750768, DOI: 10.1515/ZNC-2009-11-1211
- SCHMEDA-HIRSCHMANN, GUILLERMO ET AL: "Gastroprotective effect and cytotoxicity of natural and semisynthetic labdane diterpenes from Araucaria araucana resin", ZEITSCHRIFT FUER NATURFORSCHUNG, C: JOURNAL OF BIOSCIENCES, vol. 60, no. 7/8, 2005, pages 511-522, XP002750769,
- JUNG, HYUN AH ET AL: "Cholinesterase and BACE1 inhibitory diterpenoids from Aralia cordata", ARCHIVES OF PHARMACAL RESEARCH, vol. 32, no. 10, 2009, pages 1399-1408, XP002750770, DOI: 10.1007/S12272-009-2009-0
- LEE, MI KYEONG ET AL: "Antifibrotic activity of diterpenes from Biota orientalis leaves on hepatic stellate cells", ARCHIVES OF PHARMACAL RESEARCH, vol. 31, no. 7, 2008, pages 866-871, XP002750771, DOI: 10.1007/S12272-001-1239-9
- REN, X.-Y ET AL.: 'Labdane Diterpenes from the Seeds of Platycladus Orientalis' JOURNAL OF ASIAN NATURAL PRODUCTS RESEARCH vol. 8, no. 8, 2006, pages 677 - 682, XP055175752
- WANG, YAZHOU ET AL.: 'Diterpenoids from the Pericarp of Platycladus Orientalis' PHYTOCHEMISTRY vol. 69, no. 2, 2008, pages 518 - 526, XP022408916
- KUO,YUEHHSIUNG ET AL.: 'Chemical Constituents of the Pericarp of Platycladus Orientalis' JOURNAL OF THE CHINESE CHEMICAL SOCIETY (TAIPEI) vol. 46, no. 5, 1999, pages 819 - 824, XP055175755
- KOO, K. A. ET AL.: 'Pinusolide and 15-Methoxypinusolidic Acid Attenuate the Neurotoxic Effect of Staurosporine in Primary Cultures of Rat Cortical Cells' BRITISH JOURNAL OF PHARMACOLOGY vol. 150, no. 1, 2007, pages 65 - 71, XP055175756
- INOUE, MASANORI ET AL.: 'Terpenoids from the Seed of Platycladus Orientalis' PHYTOCHEMISTRY vol. 24, no. 7, 1985, pages 1602 - 1604, XP026680023
- KUO, YUEH-HSIUNG ET AL.: 'Four New Terpenes from the Pericarp of Platycladus Orientalis' CHEMICAL & PHARMACEUTICAL BULLETIN vol. 48, no. 6, 2000, pages 766 - 768, XP055175759
- ASILI, JAVAD ET AL.: 'Labdanes and Isopimaranes from Platycladus Orientalis and Their Effects on Erythrocyte Membrane and on Plasmodium Falciparum Growth in the Erythrocyte Host Cells' JOURNAL OF NATURAL PRODUCTS vol. 67, no. 4, 2004, pages 631 - 637, XP055175764
- CHEMICAL ABSTRACTS, 14 July 2009, Columbus, Ohio, US; abstract no. 152:223300, XP055191708 & STARK, S. ET AL.: 'Composition of Lipophilic Compounds and Carbohydrates in the Accumulated Plant Litter and Soil Organic Matter in Boreal Forests' EUROPEAN JOURNAL OF SOIL SCIENCE vol. 63, no. 1, 01 February 2012, pages 65 - 74 & DATABASE REGISTRY [Online] Database accession no. 946416-19-4
- LI, CHUANG-JUN ET AL.: 'Bis-Sesquiterpenes and Diterpenes from Chloranthus Henryi' PHYTOCHEMISTRY vol. 69, no. 16, 2008, pages 2867 - 2874, XP025685618
- CHANG, CHI-I. ET AL.: 'A New Labdane-Type Diterpene from the Bark of Juniperus Chinensis Linn.' NATURAL PRODUCT RESEARCH vol. 22, no. 13, 2008, pages 1158 - 1162, XP055175779
- ALVAREZ-MANZANEDA ROLDAN ET AL.: 'A New Route toward 7-Oxo-13-Hydroxy-8,11,13-Podocarpatrienes from Labdane Diterpenes' JOURNAL OF NATURAL PRODUCTS vol. 69, no. 4, 2006, pages 563 - 566, XP055175779
- SU, WEN-CHIUNG ET AL.: 'Labdanes from Cryptomeria Japonica' PHYTOCHEMISTRY vol. 37, no. 4, 1994, pages 1109 - 1114, XP026685839
- RODRIGUES-FILHO, EDSON ET AL.: 'Hydroxylation of the Labdane Diterpene Cupressic Acid by Fusarium Graminearum' JOURNAL OF THE BRAZILIAN CHEMICAL SOCIETY vol. 13, no. 2, 2002, pages 266 - 269, XP055175790
- NIE, JING ET AL.: 'Comparison of Modified CoMFAs in Study of PAF Antagonist' ACTA CHIMICA SINICA vol. 61, no. 7, 2003, pages 1129 - 1135, XP008175459
- BARRERO, ALEJANDRO F. ET AL.: 'Biomimetic Synthesis of 12-Oxypimaranes from 12, 13-Epoxylabdadienes' TETRAHEDRON vol. 47, no. 25, 1991, pages 4441 - 4456, XP026614947
- CALDERON, JOSE S. ET AL.: 'Labdane Diterpenes from Brickellia Glomerata' PHYTOCHEMISTRY vol. 26, no. 9, pages 2639 - 2641, XP026605097
- CAPUTO, ROMUALDO ET AL.: 'XXXII/ Diterpenoids of Araucariaceae. VII. Minor Diterpenes from e Resin of Araucaria Imbricata' GAZZETTA CHIMICA ITALIANA vol. 106, no. 11-12, 1976, pages 1119 - 1121, XP008175458
- MILLS, J. S. ET AL.: 'Revised Structure of Methyl Sciadopate' TETRAHEDRON vol. 30, no. 22, 1974, pages 4021 - 4023, XP055175831

## Description

### Technical Field:

The present invention relates to labdane diterpenoid compounds, semen boitae (*Semen boitae*) extracts as well as preparation methods and uses of the same.

### Background Arts:

Neuro degenerative diseases (Neuro degenerative diseases, NDD) are a type of chronic and progressive disease, and primarily include Alzheimer's disease (Alzheimer's disease, AD), Pakinson's disease (Pakinson's disease, PD), Huntington disease (Huntington disease, HD), Amyotrophic lateral sclerosis (Amyotrophic lateral sclerosis, ALS), and the like. This type of diseases has complex pathological and physiological process, and they have different lesion regions and causes. However, they are in common in degenerative lesions of nerve cells. Patients suffering from neuro degenerative diseases often suffer from memory deterioration, cognitive difficulties, dementia, motion balance disorder, loss of exercise ability and the like. Moreover, such patients suffer from serious decline of quality of life, long course of diseases, and impossibility of cure, which bring huge economic and psychological pressure to the patients and the family. Diseases relating to aging now become a major challenge to scientists with the global increasing aging degree.

By now, there is no measure to effectively control the progress of this type of disease in clinic. Currently, most of the drugs for treating neuro degenerative diseases only alleviate the symptoms rather than cure the diseases, and they have significant side effects. For example, by now there is no drug or treating measure which completely blocks the progressing of PD; tacrine (tacrine), drug for treating AD, has been eliminated from selection for its toxic and side effects to liver as well as significant drug-drug interactions; the side reactions of donepezil (donepezil), rivasrigmine (rivasrigmine) and galantamine (galantamine) are primarily nausea, vomiting, diarrhea and anorexia. Therefore, there is a pressing need in a drug for treating neuro degenerative diseases, which protects the nerve cells and changes the progress of the disease at the same time, and which has no side effects.

Semen boitae (*Semen boitae*), also named seed of cypress, seed of platycladusorientalis, and the like, is the mature seed of platycladusorientalis, cupressaceae family, and is primarily produced in Shandong, Henan, Hebei, Shaanxi, Hubei, Gansu and so on. Semen boitae is a frequently used Chinese traditional herb medicine. The use of semen boitae is often observed in Chinese traditional herb medicines made up of two or more ingredients, such as BaiziYangxin pills (semen boitae,codonopsis, cinnamon, polygala, schisandra, semen ziziphispinosae, cinnabar, astragalus, rhizomachuanxiong, angelica, fermented pinellia, poria, and licorice), which is used in patients lack of heart-qi and patients with insomnia or amnesia. A healthy drink for improving memory and preventing Alzheimer's disease include 21 herb medicines like semboitae, fructusrubi, poria, polygala and so on. There are few reports on the use of semen biotae extract in treating neuro degenerative diseases.

Hyun Ah Jung et al isolated fourteen diterpenes from the n-hexane fraction of the roots of *Arabia cordata* (JUNG, Hyun Ah et al: Archives of Pharmacal Research, Vol. 32, No. 10, 2009, pages 1399-1408). Through spectroscopy, the chemical structures were determined. 5 compounds were first isolated from this plant, and anti-Alzheimer and antioxidant effects were evaluated.

### Summary of the Invention:

One aim of the present invention is to provide a pharmaceutical composition, which is capable of preventing or treating neuro degenerative diseases.

The present invention provides labdane diterpenoid compounds, which includes one or more ingredients of labdane diterpenoids shown by Formula II: 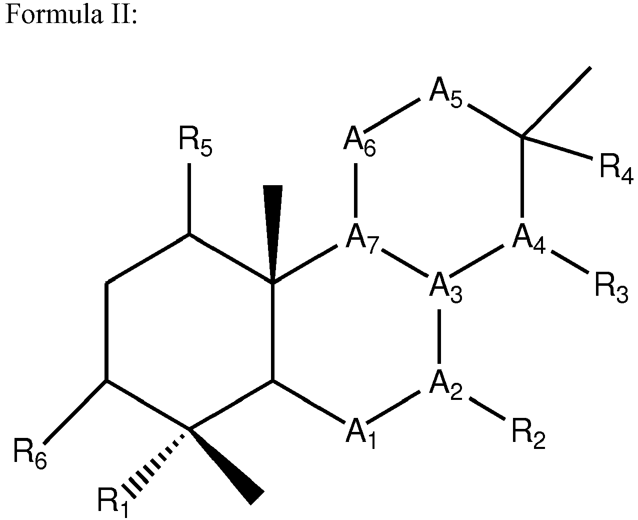
A1: 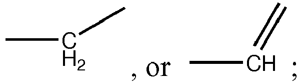
A2: 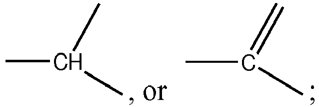
A3: 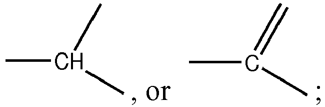
A4: 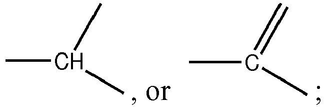
A5: 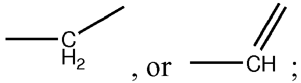
A6: 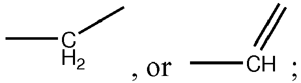
A7: 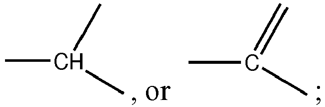
R1 : -CH₃, or -COOH, or -COOMe;
R2: -H, or -CH₂OH;
R3: 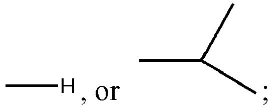
R4: 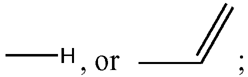
R5: -H, or =O;
R6: -H, or -OH.
for use to prevent or treat neuro degenerative diseases.

The labdane diterpenoids include 1-oxo-3β-hydroxytotarol, 6,7-dehydro-sandaracopimaric acid, sandaracopimaric acid, isopimaric acid, 7α-Hydroxysandaracopimaric acid; wherein the labdane diterpenoid compounds are used to prevent or treat neuro degenerative diseases.

The labdane diterpenoid compounds described herewith may be obtained by separating from semen boitae extracts, or obtained by derivation of precursor, semi-synthesis or total synthesis.

The present invention also provides semen boitae extracts, and said semen boitae extracts comprise one or more labdane diterpenoid compounds described above.

The present invention also provides the preparation process of semen boitae extracts:
providing powder of semen boitae;
extracting with carbon dioxide under supercritical states to remove semen boitae oil, thereby obtaining herb residue;
adding ethanol to said herb residue, and extracting the same to obtain an extractum with a relative density over 1.20;
solving said extratumin in water;
extracting with ethyl acetate to obtain the semen boitae extract. The single semen boitaediterpenoid compounds are separated by column chromatography methods.

The present invention also provides a pharmaceutical composition for use in preventing or treating neuro degenerative diseases, wherein said pharmaceutical composition comprises one or more labdane diterpenoid compounds or derivatives thereof or semen boitae extracts described above.

The present invention is further illustrated in connection with the accompanying figures and specific embodiments. However, the present invention is not limited within these embodiments. Any improvement or alteration made on the basis of the principle of the present invention also belongs to the scope of the claims of the present invention.

### Description of the Accompanying Figures

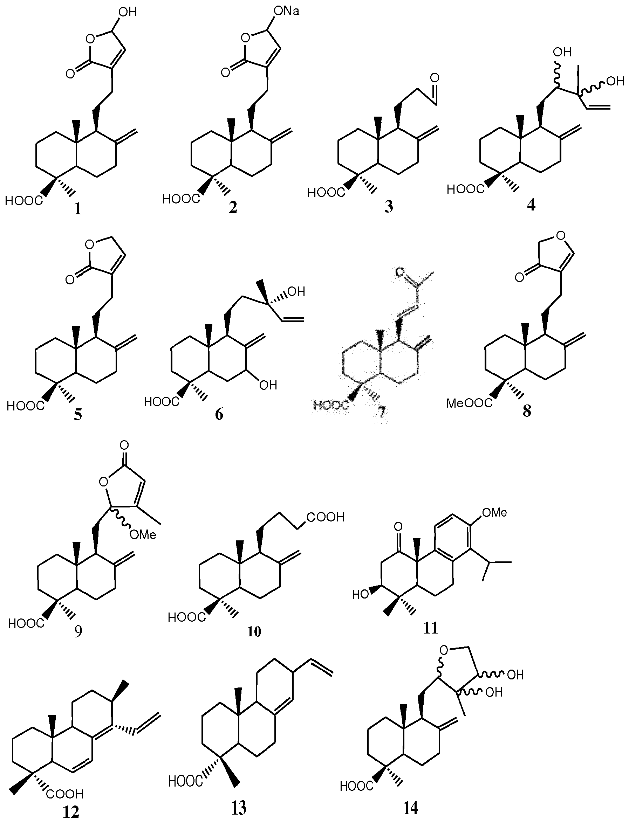

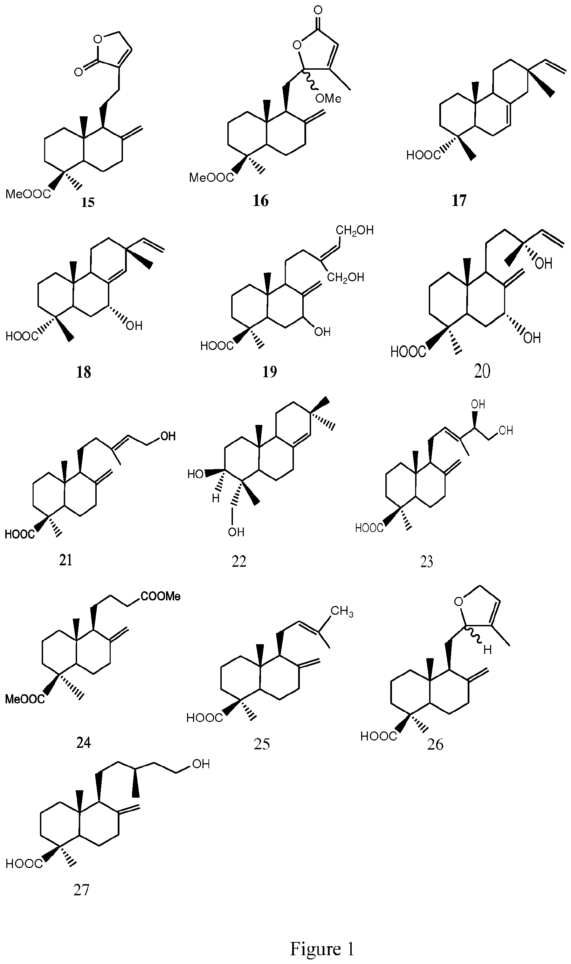

Reference Figure 1 shows the chemical formula of 26 labdanediterpenoid compounds; the names of each labdanediterpenoid compound are shown below:

| Serial | Name |
|---|---|
| 1 | 15-Hydroxypinusolidic acid |
| 2 | 15-Methoxypinusolidic acid |
| 3 | 1-Naphthalenecarboxylic acid, decahydro -1,4a-dimethyl-6-methylene-5-(3-oxobutyl)-, (1S,4aR,5S,8aR)- |
| 4 | 12,13-Dihydroxylabda-8(17),14-dien-19-oicacid |
| 5 | Pinusolidic acid |
| 6 | 7β,13S-dihydroxylabda-8(17),14-dien-19-oic acid |
| 7 | 1-Naphthalenecarboxylic acid, decahydro-1,4a-dimethyl-6-methylene-5-[(1E)-3-oxo-1-butenyl]-, (1S,4aS,5S,8aR)- |
| 8 | Isopinusolide |
| 9 | Platyclolactonic Acid |
| 10 | 14,15-bisnor-8(17)-labdene-16,19-dioic acid |
| 11 | 1-oxo-3β-hydroxytotarol |
| 12 | 6,7-Dehydrosandarapimaric Acid |
| 13 | Sandaracopimaric acid |
| 14 | 12,13,14-trihydroxylabda-12,15-epoxy-8(17)-en-19-oic acid |
| 15 | Pinusolide |
| 16 | Platyclolactonic Acid Methyl Ester |
| 17 | Isopimaric acid |
| 18 | 7α-Hydroxysandaracopimaric acid |
| 19 | 15,16-Dihydroxy-8(17),13(E)-labdadien-19-oic acid |
| 20 | 13-Epicupressic acid |
| 21 | Isocupressic acid |
| 22 | Sandaracopimaradiene-3β,18-diol |
| 23 | 14(R),15-Dihydroxy-8(17),12(E)-labdadien-19-oic acid |
| 24 | 14,15-Bisnor-8(17),12E-labdadien-19-oic acid methyl ester |
| 25 | Naphthalenecarboxylic acid, decahydro-1,4a-dimethyl-6-methylene-5-[(2E)-3-methyl-4-oxo-2-butenyl]-, (1S,4aR,5S,8aR)- |
| 26 | 16-Methyl-12,15-epoxy-8(17),13-labdadien-19-oic acid |
| 27 | Imbricatolic acid |

### Embodiments

The present invention gains significant advances in developing drugs for preventing or treating neuro degenerative diseases. Different experiments prove that semen boitae extracts and labdane diterpenoid compounds may significantly increase the activity of PD model cells, delay the paralysis time of AD model nematode, elongate the life of nematode, and delay the aging of neuroprotective activities.

### Example 1: Preparation of Semen Boitae Extracts

Obtaining semen boitae and mashing the sample. Extracting the sample with carbon dioxide under supercritical states to remove semen boitae oil. The herb residue is extracted with 95% ethanol, and each time 10 equivalent volume 95% ethanol is added. The mixture is heated to reflux, and then recover solvent from the mixture to obtain extractum with a relative density over 1.20. The extractum is solved in water, and the obtained mixture is extracted with ethyl acetate for three times. Then recover solvent from the extract liquor to obtain semen boitaediterpene extracts. Semen boitaediterpenoid compounds are obtained by separating with column chromatography.

### Example 2: Anti-PD Model Cell Experiments of Semen Boitae Extracts and Diterpene Compounds

The semen boitae extracts and semen boitaediterpenoid compounds in Example 1 are used in the experiments.

PC 12 cells (rat adrenal pheochromocytoma cell strain), which show many similar characters with dopaminergic neurons, is cultured in DMEM medium (which contains 5% horse serum, 5% fetal bovine serum, 100 U/mLpenicillin,100 g/mL streptomycin), in an incubator under 37°C, saturated humidity, and 5% CO₂. The medium is replaced every 2-3 days, and the cells are subcultured when the cells cover 70-80% area of the plate. Cells in log phase and under fine growth conditions are chosen, and inoculated in 96 well plate at 1-5 × 10⁵ and 100µl per well. After culturing under 37°C for 24 hours and the cells adhere to the walls of the wells, MPP⁺ medium is added to the model group, a medium containing MPP⁺ and semen boitae extracts/diterpenoid compounds is added to experimental group, and a medium with no drug is added to the blank control group. Then continue to culture the media in the incubator for 24 hours. MTT assay is used to evaluate cell activity.

The results are shown in Table 1, demonstrating that semen boitae extracts and diterpene compounds significantly enhance the cell activities in the PC12 cells injured by MPP⁺.

**Table 1: Effects of boitae extracts and diterpene compounds on the injuries of PC12 cells caused by MPP⁺**

| Group | average OD value | Relative Cell Activity% |
|---|---|---|
| Normal Control | 2.198±0.046 | 100 |
| Model group | 1.061±0.035 | 48.3 |
| Semen Boitae Extract | 1.543±0.066** | 70.2 |
| 15-hydroxypinusolidic acid | 1.585±0.057** | 72.1 |
| 1-Naphthalenecarboxylic acid, decahydro -1,4a-dimethyl-6-methylene-5-(3-oxobutyl)-, (1S,4aR,5S,8aR)- | 1.391±0.038* | 63.3 |
| 6,7-DehydrosandarapimaricAcid | 1.435±0.044** | 65.3 |
| 14,15-bisnor-8(17)-labdene-16,19-dioic acid | 1.292±0.072* | 58.8 |
| 1-oxo-3β-Hydroxytotarol | 1.448±0.057** | 65.9 |
| Pinusolidic acid | 1.224±0.057* | 55.7 |
| 12,13,14-trihydroxylabda-12,15-epoxy-8(17)-en-19-oi c acid | 1.404±0.057** | 63.9 |
| 7β,13S-dihydroxylabda-8(17),14-dien-19-oic acid | 1.359±0.045* | 61.8 |
| Sandaracopimaradiene-3β,18-diol | 1.652±0.015** | 75.1 |
| 14(R),15-Dihydroxy-8(17),12(E)-labdadien-19-oic acid | 1.346±0.059* | 61.2 |
| 14,15-Bisnoar-labdene-8(17),12E-dien19-oicAcid methyl Ester | 1.536±0.035** | 69.9 |
| Naphthalenecarboxylic acid, decahydro-1, 4a-dimethyl-6-methylene-5-[(2E)-3-methyl-4-oxo-2-butenyl]-, (1S,4aR,5S,8aR)- | 1.236±0.085* | 56.2 |

| | | |
|---|---|---|
| *means that P<0.05 comparing with the model group; ** means P<0.01 comparing with the model group. | | |

### Example 3: Anti-PD Model Elegan Experiments of Semen Boitae Extracts and Diterpenoid Compounds

The semen boitae extracts and semen boitaediterpenoid compounds in Example 1 are used in the experiments.

Caenorhaditiselegans (*Caenorhaditiselegans*) transgene strain CL4176 (expressing Aβ protein under temperature inducing) are synchronized and cultured under 15°C to L1 phase, and then added into coated OP50 microbial medium dishes containing the extracted drugs with different concentrations, at around 25/dish. Three dishes are used in one group. The dishes are cultured under 15°C for 12 hours and then cultures under 26°C. 36 hours after the temperature is changed, the paralyses of the elegans are observed. Count every three hours until all the elegans are paralyzed. The results are shown in the table below, demonstrating that semen boitae extracts and diterpenoid compounds both significantly delay the paralysis of CL 4176 elegan caused by Aβ.

**Table 2: Effect of semen boitae extracts and diterpenoid compounds on the paralysis of AD model elegans.**

| Group | Average Living Time (h) |
|---|---|
| Blank Control | 49.503±0.445 |
| Semen boitae extracts | 54.823±0.377** |
| pinusolide | 54.003±0.287** |
| Platyclolactonic Acid Methyl Ester | 51.393±0.487* |
| Isopimaric acid | 52.785±0.432* |
| 7α-Hydroxysandaracopimaric acid | 53.067±0.455** |
| 15,16-Dihydroxy-8(17),13(E)-labdadien-19-oic acid | 51.217±0.397* |
| 13-Epicupressic acid | 54.015±0.432** |
| Isocupressic acid | 53.879±0.414** |
| 15-Methoxypinusolidic acid | 55.012±0.399** |
| Sandaracopimaric acid | 53.616±0.443** |
| Isopinusolide | 53.988±0.320** |
| 12,13-Dihydroxylabda-8(17),14-dien-19-oicacid | 51.008±0.434* |
| 15-Hydroxypinusolidic acid | 51.575±0.257* |
| 1-Naphthalenecarboxylic acid, decahydro -1, 4a-dimethyl-6-methylene-5-(3-oxobutyl)-, (1S,4aR,5S,8aR)- | 50.991±0.568* |
| 6,7-Dehydrosandarapimaric Acid | 52.435±0.484** |
| 14,15-bisnor-8(17)-labdene-16,19-dioic acid | 51.892±0.322* |
| 1-oxo-3β-hydroxytotarol | 53.478±0.357** |
| 2-butenyl]-1-naphthoic acid | 52.136±0.415* |
| 16-Methyl-12,15-epoxy-8(17),13-labdadien-19-oic acid | 54.336±0.377** |
| Imbricatolic acid | 55.857±0.428** |

| | |
|---|---|
| *Pmeans that P<0.05 comparing with the model group; ** means P<0.01 comparing with the model group. | |

### Example 4: Anti- Elegan Aging Model Experiments of Semen Boitae Extracts and Diterpenoid Compounds

The semen boitae extracts and diterpenoid compounds in Example 1 are used in the experiments.

N2 elegans are synchronized to L4 phase, and then OP50 containing boitae extracts and diterpenoid compounds with different concentrations are coated onto NGM plates. Three plates are employed in one experimental group, and each plate includes about 25 elegans. The number of live elegans is recorded every day, and the elegans are transferred into a new NGM plate when the food is used up or there is mold contamination. The experiment pends till all the elegans are dead. The results are shown in the table below, demonstrating that semen boitae extracts and diterpenoid compounds significantly increase the average lifetime of elegans.

**Table 3: Anti-Elegan Aging Experiments of Semen Boitae Extracts and Diterpenoid Compounds**

| Group | Average Lifetime (d) |
|---|---|
| Blank Control | 13.786±0.512 |
| Semen Boitae Extracts | 17.219±0.355** |
| Sandaracopimaradiene-3β,18-diol | 14.652±0.355* |
| 14(R),15-Dihydroxy-8(17),12(E)-labdadien-19-oic acid | 16.046±0.459* |
| 14,15-Bisnor-8(17),12E-labdadien-19-oic acid methyl ester | 15.536±0.435* |
| Naphthalenecarboxylic acid, decahydro-1, 4a-dimethyl-6-methylene-5-[(2E)-3-methyl-4-oxo-2-butenyl]-, (1S,4aR,5S,8aR)- | 16.136±0.375** |
| 16-methyl-12,15-epoxyabda-8(17)-13-dien-19-oic acid | 16.336±0.370** |
| Imbricatolic acid | 15.857±0.428* |
| Platyclolactonic Acid | 16.432±0.501** |
| 1-Naphthalenecarboxylic acid, decahydro-1, 4a-dimethyl-6-methylene-5-[(1E)-3-oxo-1-butenyl]-, (1S,4aS,5S,8aR)- | 15.896±0.375* |
| pinusolide | 17.003±0.487** |
| Platyclolactonic Acid Methyl Ester | 16.384±0.387** |
| Isopimaric acid | 16.765±0.442** |
| 7α-Hydroxysandaracopimaric acid | 16.076±0.457* |
| 15,16-Dihydroxy-8(17),13(E)-labdadien-19-oic acid | 15.717±0.396* |
| 13-Epicupressic acid | 15.045±0.352** |
| Isocupressic acid | 14.889±0.414* |
| 15-Methoxypinusolidic acid | 17.018±0.389** |
| Sandaracopimaric acid | 16.716±0.449** |
| Isopinusolide | 15.928±0.370* |
| 12,13-Dihydroxylabda-8(17),14-dien-19-oicacid | 14.608±0.414* |

| | |
|---|---|
| *means that P<0.05 comparing with the model group; ** means P<0.01 comparing with the model group. | |

## Claims

1. A labdane diterpenoid compond, **characterized in that**, it has a structure represented by Formula II below: 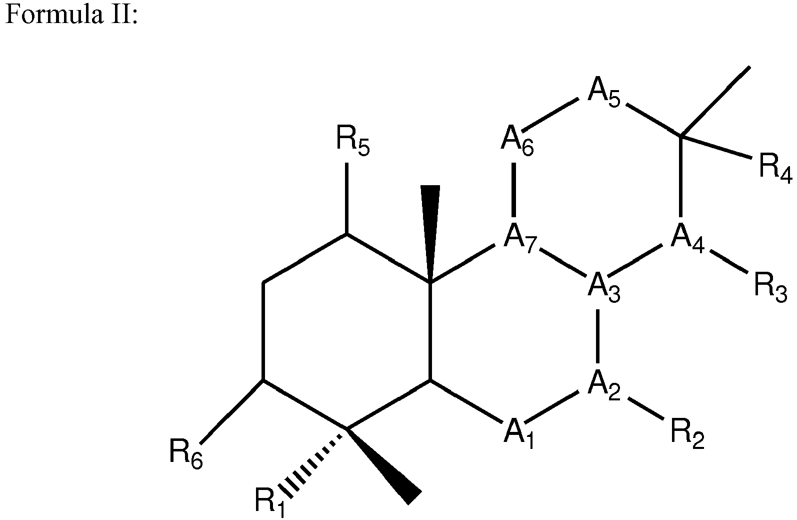
A1: 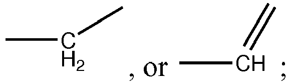
A2: 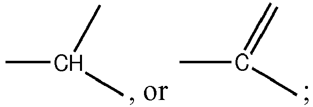
A3: 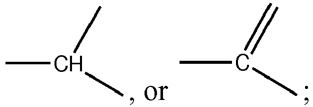
A4: 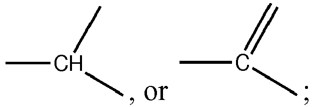
A5: 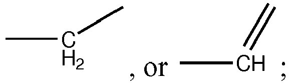
A6: 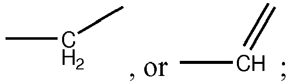
A7: 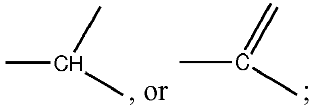
R1 : -CH₃, or COOH or -COOMe;
R2: -H, or -CH₂OH;
R3: 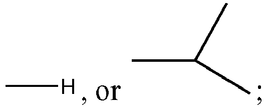
R4: 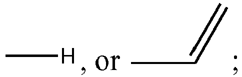
R5: -H, or =O;
R6: -H, or -OH; and
for use to prevent or treat neuro degenerative diseases.

2. The labdane diterpenoid compound according to claim 1, which includes
1-oxo-3β-hydroxytotarol,
6,7-dehydro-sandaracopimaric acid,
sandaracopimaric acid,
isopimaric acid,
7α-Hydroxysandaracopimaric acid.

3. The labdanediterpenoid compound according to claim 1, wherein the labdanediterpenoid compounds are obtained by separating from semen boitae extracts, **characterized in that**, the preparation process of semen boitae extracts include:
providing powder of semen boitae;
extracting with carbon dioxide under supercritical states to remove semen boitae oil, thereby obtaining herb residue;
adding ethanol to said herb residue, and extracting the same to obtain an extractum with a relative density over 1.20;
solving said extratumin in water;
extracting with ethyl acetate to obtain the semen boitae extract. The single semen boitaediterpenoid compounds are separated by column chromatography methods.

4. A semen boitae extract, **characterized in that**, said semen boitae extract comprise one or more labdane diterpenoid compounds in claim 1.

5. The semen boitae extract according to claim 4, **characterized in that**, the preparation process of semen boitae extracts include:
providing powder of semen boitae;
extracting with carbon dioxide under supercritical states to remove semen boitae oil, thereby obtaining herb residue;
adding ethanol to said herb residue, and extracting the same to obtain an extractum with a relative density over 1.20;
solving said extratum in water;
extracting with ethyl acetate to obtain the semen boitae extract. The single semen boitaediterpenoid compounds are separated by column chromatography methods.

6. A pharmaceutical composition for use in preventing or treating neuro degenerative diseases, **characterized in that**, said pharmaceutical composition comprises one or more labdanediterpenoid compounds in claim 1.

7. The pharmaceutical composition according to claim 6, **characterized in that**, said one or more labdanediterpenoid compounds derive from semen boitae extracts.

8. The pharmaceutical composition according to claim 6, **characterized in that**, said pharmaceutical composition is in the form of pills, powder, tablets, capsules, granules or injection dosages.

## Patentansprüche

1. Labdan-Diterpenoidverbindung, **dadurch gekennzeichnet, dass** sie eine Struktur aufweist, die durch Formel II unten dargestellt ist: 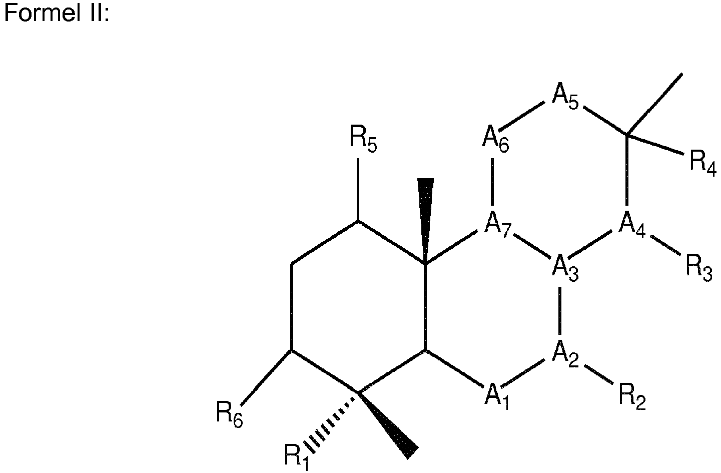
A1: 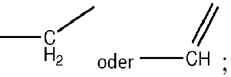
A2: 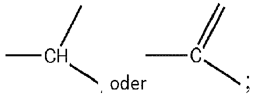
A3: 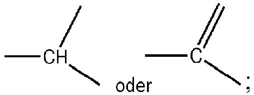
A4: 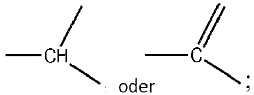
A5: 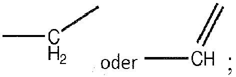
A6: 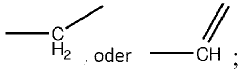
A7: 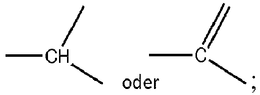
R1: -CH₃ oder -COOH oder -COOMe;
R2: -H oder -CH₂OH;
R3: 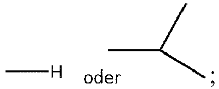
R4: 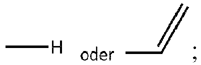
R5: -H oder =O;
R6: -H oder -OH;
zur Benutzung zum Vorbeugen gegen oder Behandeln von neurodegenerativen Erkrankungen.

2. Labdan-Diterpenoidverbindung nach Anspruch 1, die beinhaltet:
1-Oxo-3β-hydroxytotarol,
6,7-Dehydrosandaracopimarsäure,
Sandaracopimarsäure,
Isopimarsäure,
7α-Hydroxysandaracopimarsäure.

3. Labdan-Diterpenoidverbindung nach Anspruch 1, wobei die Labdan-Diterpenoidverbindungen durch Abtrennen aus Extrakten aus Boitaesamen gewonnen sind, **dadurch gekennzeichnet, dass** das Herstellungsverfahren von Extrakten aus Boitaesamen beinhaltet:
Bereitstellen von Pulver von Boitaesamen;
Extrahieren mit Kohlenstoffdioxid unter überkritischen Zuständen, um Boitaesamenöl zu entfernen, wodurch ein Kräuterrückstand gewonnen wird;
Zugeben von Ethanol zu dem Kräuterrückstand und Extrahieren desselben, um ein Extractum mit einer relativen Dichte von über 1,20 zu gewinnen;
Lösen des Extractums in Wasser;
Extrahieren mit Ethylacetat, um den Extrakt aus Boitaesamen zu gewinnen. Die einzelnen Diterpenoidverbindungen des Boitaesamens werden mithilfe säulenchromatographischer Verfahren getrennt.

4. Extrakt aus Boitaesamen, **dadurch gekennzeichnet, dass** der Extrakt aus Boitaesamen eine oder mehrere Labdan-Diterpenoidverbindungen von Anspruch 1 umfasst.

5. Extrakt aus Boitaesamen nach Anspruch 4, **dadurch gekennzeichnet, dass** das Herstellungsverfahren von Extrakten aus Boitaesamen beinhaltet:
Bereitstellen von Pulver von Boitaesamen;
Extrahieren mit Kohlenstoffdioxid unter überkritischen Zuständen, um Boitaesamenöl zu entfernen, wodurch ein Kräuterrückstand gewonnen wird;
Zugeben von Ethanol zu dem Kräuterrückstand und Extrahieren desselben, um ein Extractum mit einer relativen Dichte von über 1,20 zu gewinnen;
Lösen des Extractums in Wasser;
Extrahieren mit Ethylacetat, um den Extrakt aus Boitaesamen zu gewinnen. Die einzelnen Diterpenoidverbindungen des Boitaesamens werden mithilfe säulenchromatographischer Verfahren getrennt.

6. Pharmazeutische Zusammensetzung zur Benutzung zum Vorbeugen gegen oder Behandeln von neurodegenerativen Erkrankungen, **dadurch gekennzeichnet, dass** die pharmazeutische Zusammensetzung eine oder mehrere Labdan-Diterpenoidverbindungen von Anspruch 1 umfasst.

7. Pharmazeutische Zusammensetzung nach Anspruch 6, **dadurch gekennzeichnet, dass** die eine oder mehreren Labdan-Diterpenoidverbindungen aus Extrakten aus Boitaesamen stammen.

8. Pharmazeutische Zusammensetzung nach Anspruch 6, **dadurch gekennzeichnet, dass** die pharmazeutische Zusammensetzung in der Form von Pillen, Pulver, Tabletten, Kapseln, Körnchen oder Injektionsdosierungen ist.

## Revendications

1. Composé labdane diterpénoïde, **caractérisé en ce que**, il a une structure représentée par la formule II ci-dessous : 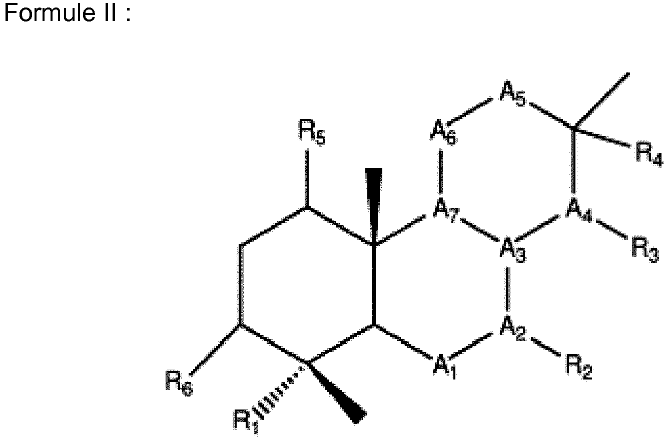
A1: 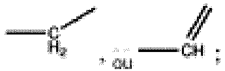
A2: 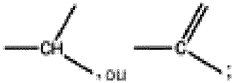
A3: 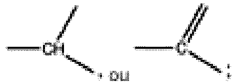
A4: 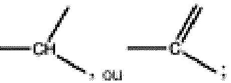
A5: 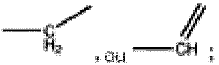 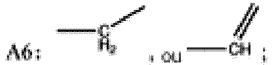 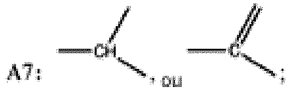
R1: -CH₃, ou -COOH, ou -COOMe;
R2: -H, ou -CH₂CH; 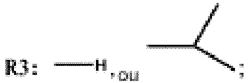 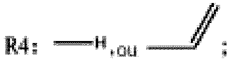
R5: -H, ou =O;
R6: -H, ou -OH; et
pour une utilisation dans le traitement ou la prévention de maladies neurodégénératives.

2. Composé labdane diterpénoïde selon la revendication 1, qui comprend
le 1-oxo-3b-hydroxytotarol,
l'acide 6,7-déhydro-sandaracopimarique,
l'acide sandaracopimarique,
l'acide isopimarique,
l'acide 7a-hydroxysandaracopimarique.

3. Composé labdane diterpénoïde selon la revendication 1, dans lequel les composés labdane diterpénoïdes sont obtenus par séparation d'extraits de Semen boitae, **caractérisés en ce que**, le procédé de préparation des extraits de semen boitae comprend :
la fourniture de poudre de semen boitae,
l'extraction avec du dioxyde de carbone dans des états surcritiques pour enlever l'huile de semen boitae, obtenant ainsi le résidu d'herbe ;
l'addition d'éthanol audit résidu d'herbe, et extraction de celui-ci pour obtenir un extrait avec une densité relative supérieure à 1,20 ;
la solubilisation dudit extrait dans de l'eau,
l'extraction avec de l'acétate d'éthyle pour obtenir l'extrait de semen boitae. Les simples composés diterpénoïdes de semen boitae sont séparés par des procédés de chromatographie sur colonne.

4. Extrait de semen boitae, **caractérisé en ce que**, ledit extrait de semen boitae comprend un ou plusieurs composés labdane diterpénoïdes selon la revendication 1.

5. Extrait de semen boitae selon la revendication 4, **caractérisé en ce que**, le procédé de séparation des extraits de semen boitae comprend :
la fourniture de poudre de semen boitae,
l'extraction avec du dioxyde de carbone dans des états surcritiques pour enlever l'huile de semen boitae, obtenant ainsi le résidu d'herbe ;
l'addition d'éthanol audit résidu d'herbe, et extraction de celui-ci pour obtenir un extrait avec une densité relative supérieure à 1,20 ;
la solubilisation dudit extrait dans de l'eau,
l'extraction avec de l'acétate d'éthyle pour obtenir l'extrait de semen boitae. Les simples composés diterpénoïdes de semen boitae sont séparés par des procédés de chromatographie sur colonne.

6. Composition pharmaceutique pour une utilisation dans la prévention ou le traitement de maladies neurodégénératives, **caractérisée en ce que**, ladite composition pharmaceutique comprend un ou plusieurs composés labdane diterpénoïdes selon la revendication 1.

7. Composition pharmaceutique selon la revendication 6, **caractérisée en ce que**, lesdits un ou plusieurs composés labdane terpénoïdes sont dérivés d'extraits de semen boitae.

8. Composition pharmaceutique selon la revendication 6, **caractérisée en ce que**, ladite composition pharmaceutique est sous forme de pilules, de poudre, de comprimés, de capsules, de granulés ou de doses d'injection.
